(19) Europäisches Patentamt
European Patent Office
Office européen des brevets

(11) Veröffentlichungsnummer: **0 046 859**
**B1**

# (12) EUROPÄISCHE PATENTSCHRIFT

(45) Veröffentlichungstag der Patentschrift: **06.07.83**

(51) Int. Cl.³: **C 07 C 25/02**, C 07 C 87/60, C 07 C 85/24, C 07 C 85/20, C 07 C 103/375, C 07 C 102/00

(21) Anmeldenummer: **81105557.3**

(22) Anmeldetag: **15.07.81**

## (54) Verfahren zur Herstellung von 1-Brom-3,5-dichlorbenzol.

(30) Priorität: **03.09.80 DE 3033122**

(43) Veröffentlichungstag der Anmeldung: **10.03.82 Patentblatt 82/10**

(45) Bekanntmachung des Hinweises auf die Patenterteilung: **06.07.83 Patentblatt 83/27**

(84) Benannte Vertragsstaaten: **BE CH DE FR GB IT LI**

(56) Entgegenhaltungen:
**ARKIV FOR KEMI, Band 21, Nr. 28, 8. November 1963, Seiten 301–308 Stockholm, SE. T. RAZNIKIEWICZ: »On the synthesis of alpha-methoxy-2,4-dichlorophenyl-acetic acid and alpha-methoxy-3,5-dichlorophenylacetic acid«**

(73) Patentinhaber: **BASF Aktiengesellschaft, Carl-Bosch-Strasse 38, D-6700 Ludwigshafen (DE)**

(72) Erfinder: **Jersak, Ulrich, Dr., Dubliner Strasse 21, D-6700 Ludwigshafen (DE)**
Erfinder: **Scheuermann, Horst, Dr., Bexbacher Strasse 41, D-6700 Ludwigshafen (DE)**

EP 0 046 859 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 1-Brom-3,5-dichlorbenzol

Die Erfindung betrifft ein Verfahren zur Herstellung von 1-Brom-3,5-dichlorbenzol durch Umsetzung von Acetanilid mit Chlor bei einer Temperatur unterhalb 35°C in Gegenwart von 40- bis 70gewichtsprozentiger, wäßriger Schwefelsäure und Behandlung des so gebildeten Dichlorphenylacetamids oberhalb 75°C, dann durch Umsetzung mit Brom bei einer Temperatur von 60 bis 120°C und schließlich durch Abspaltung der Aminogruppe von dem so erhaltenen Monobromdichloranilin.

In der deutschen Offenlegungsschrift 2 737 797 wird beschrieben, daß man 1-Brom-3,5-dichlorbenzol durch eine spezielle Isomerisierung von anderen Monobromdichlorbenzolen in Anwesenheit oder Abwesenheit von Dichlorbenzol und Dibromdichlorbenzol bei 80 bis 180°C in Anwesenheit von Aluminiumhalogenid bei einem Molverhältnis von 0,02 bis 1, bezogen auf die Gesamtmenge der Halogenbenzole, erhält. Danach wird das gebildete 1-Brom-3,5-dichlorbenzol vom Reaktionsgemisch abgetrennt und der Rückstand unter Vermischung mit frischem Ausgangsmaterial zurückgeführt. Nachteilig sind die Verwendung schwer zugänglicher Produkte wie 1,3-Dichlorbenzol, die Verwendung von Aluminiumhalogenid und Dihalogenbenzolen in speziellen Mengen und die aufwendige Aufarbeitung.

Ein weiteres Verfahren benutzt das einfach zugängliche Acetanilid als Ausgangsprodukt (Arkiv Kemi 21, 301 bis 307 [1963]). Zuerst wird in Eisessig/Natriumacetat Acetanilid zu N-2,4-Dichlorphenylacetamid chloriert. Das bei der Chlorierung entstandene NaCl wird durch Filtration abgetrennt. Das Filtrat wird mit konzentrierter Salzsäure angesäuert und am Rückfluß gekocht. Nun wird zum Reaktionsgemisch Brom hinzugetropft, wobei 2-Brom-4,6-dichloranilin entsteht. Nicht umgesetztes Brom wird mit Natriumbisulfit zerstört. Dieses Anilinderivat wird ohne Zwischenisolierung bei 0°C diazotiert. Die Umsetzung des Diazoniumsalzes mit überschüssigem Natriumhypophosphit liefert das gewünschte 1-Brom-3,5-dichlorbenzol in einer Gesamtausbeute von 54 Prozent.

Es wurde nun gefunden, daß man 1-Brom-3,5-dichlorbenzol vorteilhaft erhält, wenn man

a) Acetanilid bei einer Temperatur unterhalb 35°C in Gegenwart von 40- bis 70gewichtsprozentiger, wäßriger Schwefelsäure mit Chlor umsetzt,
b) die so gebildeten Dichlorphenylacetamide bei einer Temperatur oberhalb 75°C bis 150°C entacetylisiert,
c) dem Reaktionsgemisch bei einer Temperatur von 60°C bis 120°C Brom zugibt und
d) von dem so erhaltenen Monobromdichloranilin in bekannter Weise die Aminogruppe abspaltet.

Die Umsetzung wird durch die folgenden Formeln wiedergegeben, wobei nur in Form eines Beispiels der prozentuale Mengenanteil und Zusammensetzung der Isomerengemische angegeben werden:

a) 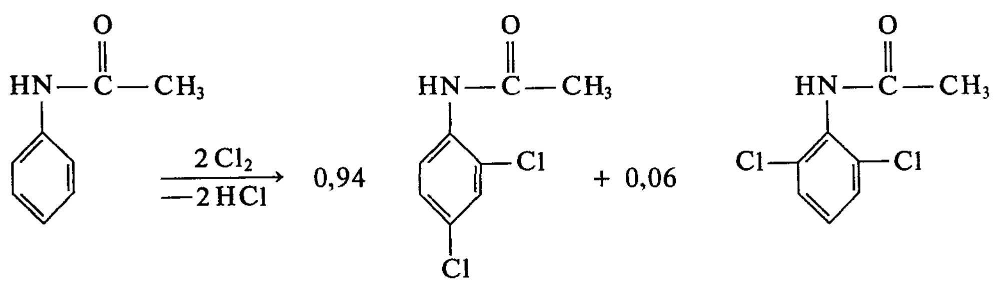


b) 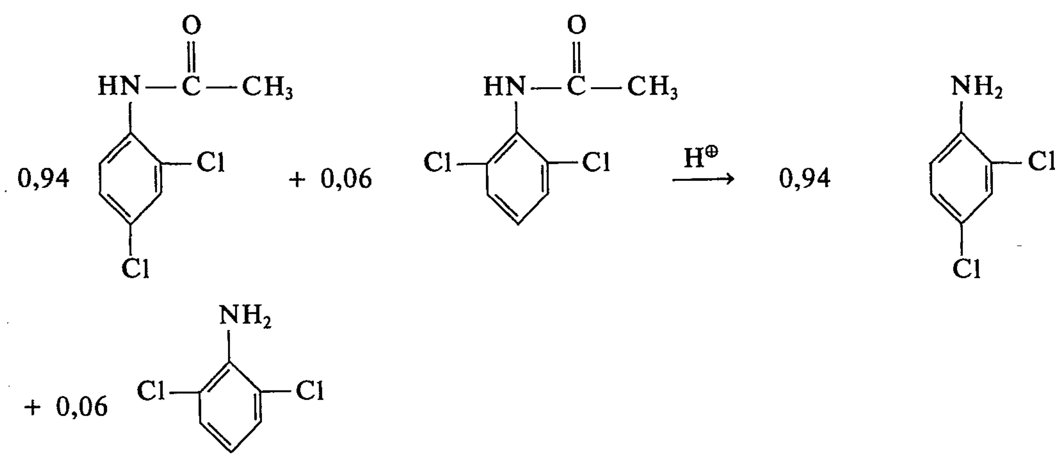

c) 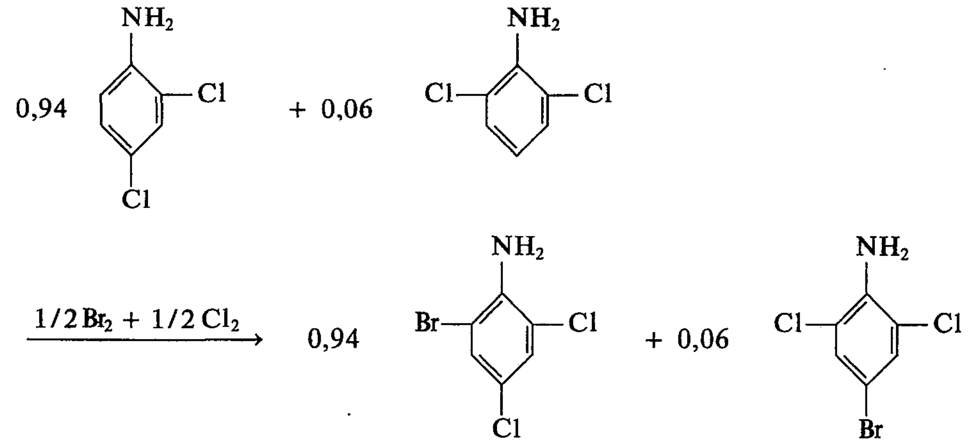


d) 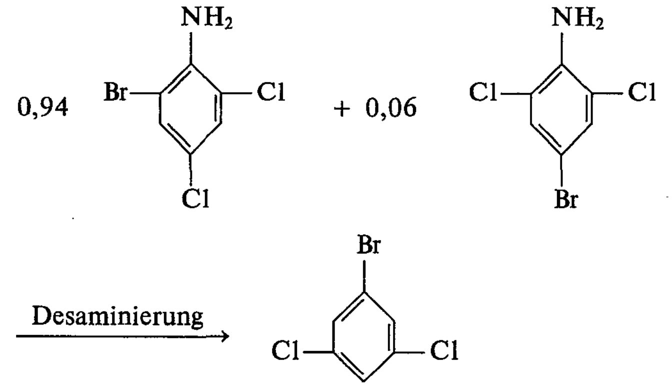


Im Hinblick auf die bekannten Verfahren liefert das Verfahren nach der Erfindung auf einfacherem und wirtschaftlicherem Wege 1-Brom-3,5-dichlorbenzol in besserer Ausbeute und Reinheit. Die Verwendung von gerade im großtechnischen Maßstab unwirtschaftlichen, schwer zugänglichen und betrieblich umständlich zu handhabenden Stoffen wird vermieden. Die Verwendung von teuren Stoffen wie Eisessig oder Phosphorverbindungen, die insbesondere auch die Abwässerregenerierung stören, wird vermieden. Alle diese vorteilhaften Ergebnisse sind im Hinblick auf den Stand der Technik überraschend.

Im Schritt a) (Chlorierung) des erfindungsgemäßen Verfahrens verwendet man Temperaturen unterhalb 35° C, zweckmäßig von 15 bis unterhalb 35° C, vorzugsweise von 20 bis 30° C und 40- bis 70-, vorzugsweise 50- bis 60gewichtsprozentige, wäßrige Schwefelsäure. Vorteilhaft kommt Chlor in einer Menge von 2 bis 3, insbesondere 2 bis 2,1 Mol Chlor je Mol Acetanilid in Betracht. Zweckmäßig verwendet man 6 bis 20, insbesondere 8 bis 14 Mol Schwefelsäure je Mol Acetanilid. Die Reaktionszeit in Stufe a) beträgt vorteilhaft 1 bis 4, insbesondere 2 bis 3 Stunden. In einer bevorzugten Ausführungsform legt man wäßrige Schwefelsäure und Acetanilid vor und gibt dann langsam Chlor bei vorgenannten Reaktionstemperaturen zu.

Alle Schritte a) bis d) können unter Überdruck, Unterdruck oder zweckmäßig drucklos, diskontinuierlich oder kontinuierlich, durchgeführt werden.

In der Stufe b) wird das Reaktionsgemisch auf höhere Temperatur, zweckmäßig während 30 bis 120 Minuten auf oberhalb 75 bis 150°C, vorteilhaft 100 bis 120°C, gebracht und bei dieser Temperatur während 0,5 bis 1 Stunde umgesetzt, d. h. entacetylisiert.

Zweckmäßig leitet man zwischen den Stufen a) und b) Inertgase, z. B. Stickstoff, während 20 bis 30 Minuten durch das Reaktionsgemisch, um unumgesetztes Halogen zu entfernen. Die durchgeleitete Inertgasmenge beträgt zweckmäßig 5 bis 10 Mol je Mol Acetanilid. Dann wird während 20 bis 30 Minuten das Reaktionsgemisch auf die Temperatur von b) gebracht und bei dieser Temperatur während 0,2 bis 1 Stunde umgesetzt. Man erhält im allgemeinen ein Gewichtsverhältnis von 15 N-2,4-Dichlorphenylacetamid zu 1 N-2,6-Dichlorphenylacetamid.

In der Stufe c) wird Brom gasförmig oder flüssig oder zweckmäßig auch im Gemisch mit weiteren Lösungsmitteln, z. B. Essigsäure, zugegeben. Vorteilhaft kommen 10 bis 20 Gewichtsprozent Lösungsmittel, bezogen auf die Gewichtsmenge Brom, zur Anwendung. Gegebenenfalls kann man Brom auch in situ erzeugen, indem man 50 bis 99 Molprozent der Gesamtbrommenge vorlegt und dann langsam 1 bis 50 Molprozent Chlor, bezogen auf die Gesamtmenge an Halogen, zuführt. Auf diese vorteilhafte Form wird teures Brom durch billiges Chlor ersetzt, da Brom im Reaktionsgemisch Bromwasserstoff bildet, der durch Chlor erneut in Brom wiederverwandelt wird.

Nach Stufe b) wird die Reaktionstemperatur von b) auf die Temperatur von c) eingestellt. Die Temperatur der Stufe c) beträgt 60 bis 120°C, insbesondere 70 bis 110°C, die Reaktionszeit 0,2 bis 1 Stunde. In einer bevorzugten Ausführungsform wird nun dem Reaktionsgemisch am Ende der Stufe c) 0,4 bis 0,6 Mol Chlor je Mol Dichlorphenylacetamid zugegeben und während 0,2 bis 0,5 Stunden, vorteilhaft bei einer Temperatur von 70 bis 80°C, die Reaktion beendet. Dann wird zweckmäßig wie zwischen den Stufen a) und b) auch zwischen den Stufen c) und d) Inertgas während 20 bis 30 Minuten bei 50 bis 70°C durch das Reaktionsgemisch in einer Menge von 3 bis 4 Mol Inertgas je Mol Dichlorphenylacetamid geführt. Anschließend stellt man die Temperatur auf die der Stufe d) ein. Man erhält in Stufe c) ein Gemisch von 2,4-Dichlor-6-brom-anilin und 2,6-Dichlor-4-brom-anilin, meist in einem Verhältnis von 15 Mol 2,4-Dichlor-6-brom-anilin zu 1 Mol 2,6-Dichlor-4-brom-anilin.

In der Stufe d), zweckmäßig nach Entfernung leichter siedender Gemischskomponenten durch fraktionierte Destillation, befindet sich das so erhaltene Gemisch von 2,6-Dichlor-4-brom-anilin und 2,4-Dichlor-6-brom-anilin als Destillationsrückstand und wird nun in üblicher Weise desaminiert. Die Desaminierung kann nach bekannten Arbeitsweisen durchgeführt werden (Houben-Weyl, Methoden der Organischen Chemie, Band 11/2, Seiten 216 bis 221). Man kann die Bromdichloraniline bei 20 bis 30°C in bekannter Weise diazotieren, mit z. B. Natriumnitrit und reduziert anschließend in an sich bekannter Weise bei höherer Temperatur, zweckmäßig von 30 bis 50°C in Gegenwart einer geringen Menge metallischen Kupfers mit Alkoholen (z. B. Methanol, Äthanol, Isopropanol), Ameisensäurederivaten (Dimethylformamid, $HCO_2H$), Äthern (Tetrahydrofuran, Dioxan) oder auch mit der zweckmäßig stöchiometrischen Menge Natriumhypophosphit. Man kann auch den Reaktionsansatz in Gegenwart von Isopropanol oder Methanol oder Äthanol und wenig metallischen Kupfers bei Temperaturen von zweckmäßig 40 bis 70°C mit Natriumnitrit nitrosieren.

Nach Stufe d) wird der Endstoff aus dem Reaktionsgemisch in bekannter Weise, z. B. durch Wasserdampfdestillation und Filtration, isoliert. Der Endstoff kann ebenfalls als Festprodukt oder als Schmelze durch Phasentrennung bei Temperaturen von z. B. 80°C abgetrennt werden.

Das nach dem Verfahren der Erfindung herstellbare 1-Brom-3,5-dichlorbenzol ist ein wertvoller Ausgangsstoff für die Herstellung von Farbstoffen und Schädlingsbekämpfungsmitteln. 1-Brom-3,5-dichlorbenzol ist ein Ausgangsprodukt zur Herstellung von 3,5-Dichloranilin durch Umsetzung mit Ammoniak unter Druck, das zur Synthese von Fungiziden, z. B. Vinclozolin dient.

Die in den folgenden Beispielen angeführten Teile bedeuten Gewichtsteile.

## Beispiel 1

In 2000 Teilen 55prozentiger, wäßriger Schwefelsäure werden 135 Teile Acetanilid gelöst. Bei 20 bis 30°C werden innerhalb von 2 Stunden 150 Teile Chlor eingeleitet. Man rührt 30 Minuten nach und leitet anschließend 30 Minuten lang Stickstoff durch den Reaktionsansatz. Es wird auf 120°C erwärmt und eine Stunde bei dieser Temperatur gerührt. Dann werden bei 120 auf 70°C fallender Temperatur während 15 Minuten 81 Teile Brom zugegeben und anschließend bei 75°C innerhalb einer Stunde 35 Teile Chlor eingeführt. Nach 30 Minuten unter Rühren leitet man weitere 30 Minuten lang 100 Teile Stickstoff durch die Mischung. Bei 30 mbar wird das Reaktionsgemisch bis zu einer Sumpftemperatur von 70°C destilliert; es destillieren 200 Teile Destillat ab. Zum Destillationsrückstand fügt man 300 Teile Isopropanol und 2 Teile Kupferschliff hinzu. Innerhalb von 1,5 Stunden gibt man bei 70 bis 75°C 305 Teile 25gewichtsprozentige, wäßrige $NaNO_2$-Lösung zu. Das Gemisch wird 45 Minuten bei 70°C nachgerührt und anschließend mit Wasserdampf destilliert. Das im Destillat auskristallisierte 1-Brom-3,5-dichlorbenzol wird abgesaugt und getrocknet. Man erhält 176 Teile 1-Brom-3,5-dichlorbenzol vom Schmelzpunkt 72 bis 74°C (78% der Theorie).

## Beispiel 2

In 2000 Teilen 55prozentiger wäßriger Schwefelsäure werden 135 Teile Acetanilid gelöst. Bei 20 bis 30°C werden innerhalb von 2 Stunden 150 Teile Chlor eingeleitet. Man rührt das Gemisch 30 Minuten nach und leitet anschließend 30 Minuten lang Stickstoff durch den Reaktionsansatz. Das Gemisch wird auf 120°C erwärmt und eine Stunde bei dieser Temperatur gerührt. Dann werden bei 120 auf 70°C (während 15 Minuten) fallender Temperatur 81 Teile Brom zugegeben und anschließend bei 75°C innerhalb einer Stunde 35 Teile Chlor eingeführt. Nach 30 Minuten unter Rühren leitet man weitere 30 Minuten lang 200 Teile Stickstoff durch den Ansatz. Bei 30 mbar wird das Reaktionsgemisch bis zu einer Sumpftemperatur von 70°C destilliert; es destillieren 200 Teile Destillat über. Den Destillationsrückstand kühlt man auf 25°C ab und gibt innerhalb einer Stunde 280 Teile 25gewichtsprozentige, wäßrige $NaNO_2$-Lösung zu. Man läßt das Gemisch 30 Minuten nachrühren und fügt dann 2 Teile Amidosulfonsäure hinzu. Die Lösung wird filtriert und innerhalb von 30 Minuten zu einem Gemisch von 160 Teilen Methanol + 2 Teilen Kupferpulver, das am Rückfluß siedet, hinzugefügt. Man läßt das Gemisch eine Stunde bei 75°C nachrühren und trennt anschließend die spezifisch schwerere organische Phase bei 70°C ab. Man erhält 205 Teile Rohschmelze vom

Erstarrungspunkt 58°C. Nach einmaligem Umkristallisieren aus Isopropanol erhält man 195 Teile 1-Brom-3,5-dichlorbenzol vom Schmelzpunkt 75 bis 76°C (Ausbeute 86% der Theorie).

Beispiel 3

Die Umsetzung wird analog Beispiel 2 durchgeführt. Anstelle von 160 Teilen Methanol werden 200 Teile Dimethylformamid eingesetzt. Man erhält nach der Reinigung durch Umkristallisation 190 Teile 1-Brom-3,5-dichlorbenzol vom Schmelzpunkt 74 bis 76°C (Ausbeute 84% der Theorie).

## Patentanspruch

Verfahren zur Herstellung von 1-Brom-3,5-dichlorbenzol, dadurch gekennzeichnet, daß man

a) Acetanilid bei einer Temperatur unterhalb 35°C in Gegenwart von 40- bis 70gewichtsprozentiger, wäßriger Schwefelsäure mit Chlor umsetzt, dann
b) die so gebildeten Dichlorphenylacetamide bei einer Temperatur oberhalb 75°C bis 150°C entacetylisiert,
c) dem Reaktionsgemisch bei einer Temperatur von 60°C bis 120°C Brom zugibt und
d) von dem so erhaltenen Monobromdichloranilin in bekannter Weise die Aminogruppe abspaltet.

## Claim

A process for the production of 1-bromo-3,5-dichlorobenzene, wherein

(a) acetanilide is reacted with chlorine at a temperature below 35°C in the presence of 40 to 70% strength by weight aqueous sulphuric acid,
(b) the dichlorophenyl acetamide thus formed is deacetylized at a temperature above 75°C up to 150°C,
(c) bromine is added to the reaction mixture at a temperature of from 60 to 120°C, and
(d) the amino group is eliminated in a conventional manner from the monobromodichloroaniline thus obtained.

## Revendication

Procédé de préparation du bromo-1 dichloro-3,5 benzène, caractérisé en ce que:

a) on fait réagir l'acét-anilide à une température inférieure à 35°C et en présence d'un acide sulfurique aqueux d'une concentration de 40 à 70% en poids avec du chlore;
b) on soumet les dichlorophényl-acétamides formés à une température supérieure à 75°C et pouvant aller jusqu' à 150°C à une désacétylation;
c) on ajoute au mélange réactionnel, à une température comprise entre 60 et 120°C, du brome, puis
d) on provoque de manière connue en soi le clivage du groupe amino de la monobromo-dichlor-aniline obtenue.